# EUROPEAN PATENT APPLICATION

(11) **EP 2 881 115 A1**
(43) Date of publication of application: **10.06.2015**
(21) Application number: 13825231.7
(22) Date of filing: 30.07.2013
(51) Int. Cl.: A61K 31/426, A61K 31/4439, A61K 31/444, A61K 45/00, A61P 1/04, A61P 43/00

(54) **MEDICINE AGAINST GASTROESOPHAGEAL REFLUX DISEASE**

(30) Priority: 31.07.2012 JP 2012169037; 26.12.2012 JP 2012281959
(71) Applicant: ONO Pharmaceutical Co., Ltd., Osaka-shi, Osaka 541-8526 (JP)
(72) Inventor: OKADA, Hiroki, Mishima-gun Osaka 618-8585 (JP); KONEMURA, Takashi, Mishima-gun Osaka 618-8585 (JP)
(74) Representative: Grünecker Patent- und Rechtsanwälte PartG mbB
(86) International application number: PCT/JP2013/070581
(87) International publication number: WO 2014/021306

(57) **Abstract**

The present invention provides an agent for preventing or relapse-preventing, treating or ameliorating gastroesophageal reflux disease, in which existing treatment is not effective.

4-[({6-[Isobutyl(1,3-thiazol-2-ylsulfonyl)amino]-2,3-dihydro-1H-inden-5-yl}oxy)methyl]-3-methyl benzoic acid, a salt thereof or a prodrug thereof is effective in the prevention or relapse prevention, treatment or amelioration of gastroesophageal reflux disease.

## Description

### TECHNICAL FIELD

The present invention relates to the use of 4-[({6-[isobutyl(1,3-thiazol-2-ylsulfonyl)amino]-2,3-dihydro-1H-inden-5-yl}oxy)methyl]-3-methyl benzoic acid, a salt thereof or a prodrug thereof (hereinafter, may be abbreviated as the compound of the present invention) as a therapeutic or ameliorating agent for Gastroesophageal Reflux Disease (hereinafter, may be abbreviated as GERD (Gastroesophageal Reflux Disease)).

### BACKGROUND ART

GERD is a disease which affects healthy lives by the reflux of gastric acid or stomach contents into the esophagus and is accompanied by troublesome symptoms or physical complications. The troublesome symptoms are mainly heartburn, acid regurgitation and the like, and complications such as esophagitis (reflux esophagitis) can be caused. Meanwhile, GERD unaccompanied by esophagitis has also been called Non-Erosive Reflux Disease (NERD (Non-Erosive Reflux Disease)) in recent years.

Nowadays, proton pump inhibitors, histamine H2 inhibitors, gastrointestinal prokinetic agents, Chinese medicinal drugs and the like are used for treatment of GERD. The US FDA points out that particularly proton pump inhibitors which are frequently used are related to an increase in the fracture rate and Clostridium difficile-associated diarrhea, and a warning is given that the use thereof should be limited to a low dose or a short period of time. Although there are limitations of the effects, the inhibitors are desultorily used, which has been seen as a problem. In addition, it is known that existing agents including proton pump inhibitors have weak effects on subjective symptoms of patients classified into NERD which does not have mucosal damages as compared to subjective symptoms accompanied by reflux esophagitis caused by mucosal damages. Patients in which proton pump inhibitors are not effective have less options, and the QOL (Quality Of Life) of the patients cannot be controlled sufficiently. Therefore, it is strongly demanded that novel drugs, which do not have side effects, for GERD, in particular NERD, in which existing treatment is not effective, be developed.

Incidentally, the compound of the present invention is a compound described in Example 2 (105) in Patent Document 1 and also reported in Patent Document 2 and Patent Document 3. In these documents, however, it is not suggested that the compound of the present invention is useful as an agent for GERD.

### PRIOR ART LITERATURE

### PATENT DOCUMENT

Patent Document 1: WO 2002/072564
Patent Document 2: WO 2002/072145
Patent Document 3 : WO 2008/099907

### SUMMARY OF THE INVENTION

### PROBLEMS TO BE SOLVED BY THE INVENTION

An object of the present invention is to provide an agent for treating or ameliorating gastroesophageal reflux disease.

### MEANS FOR SOLVING THE PROBLEMS

As a result of intensive investigation, the present inventors found that the above-mentioned problem is solved by the compound of the present invention, thereby completing the present invention.

That is, the present invention is as follows.
[1] A therapeutic and/or ameliorating agent for gastroesophageal reflux disease, containing 4-[({6-[isobutyl(1,3-thiazol-2-ylsulfonyl)amino]-2,3-dihydro-1H-inden-5-yl}oxy)methyl]-3-methyl benzoic acid represented by the following formula: 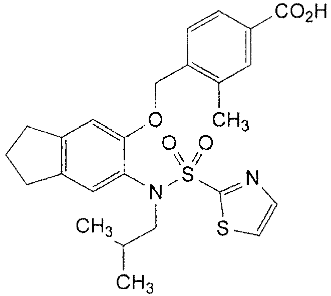 (hereinafter, may be abbreviated as Compound A), a salt thereof or a prodrug thereof as an active ingredient.
[2] The agent according to [1] above, wherein the gastroesophageal reflux disease is non-erosive.
[3] The agent according to [1] or [2] above, wherein the symptoms of the gastroesophageal reflux disease are heartburn and/or acid reflux.
[4] The agent according to any of [1] to [3] above, wherein the gastroesophageal reflux disease is resistant to a proton pump inhibitor.
[5] The agent according to any of [1] to [4] above, which is used in combination with a medicine selected from proton pump inhibitors, histamine H2 receptor antagonists, gastrointestinal prokinetic agents, oral antacid mixtures, mucosal protective agents and dopamine receptor antagonists.
[6] The agent according to any of [1] to [3] above, which is used in combination with a proton pump inhibitor.
[7] The agent according to [5] or [6] above, wherein the proton pump inhibitor is a medicine selected from omeprazole, esomeprazole, pantoprazole, rabeprazole, tenatoprazole, ilaprazole and lansoprazole.
[8] An agent for suppressing hypersensitivity in the esophagus, containing 4-[({6-[isobutyl(1,3-thiazol-2-ylsulfonyl)amino]-2,3-dihydro-1H-inden-5-yl}oxy)methyl]-3-methyl benzoic acid, a salt thereof or a prodrug thereof as an active ingredient.
[9] An agent for suppressing transient lower esophageal sphincter relaxation, containing 4-[({6-[isobutyl(1,3-thiazol-2-ylsulfonyl)amino]-2,3-dihydro-1H-inden-5-yl}oxy)methyl]-3-methyl benzoic acid, a salt thereof or a prodrug thereof as an active ingredient.
[10] The agent according to [9] above, which reduces the frequency of transient lower esophageal sphincter relaxation.
[11] The agent according to [9] or [10] above for suppressing the reflux of gastric acid and/or gastric contents.
[12] The agent according to any of [9] to [11] above for the prevention or relapse prevention, treatment and/or amelioration of gastroesophageal reflux disease.
[13] The agent according to [12] above, wherein the gastroesophageal reflux disease is non-erosive.
[14] The agent according to [12] or [13] above, wherein the symptoms of gastroesophageal reflux disease are heartburn and/or acid reflux.
[15] The agent according to any of [12] to [14] above, wherein the gastroesophageal reflux disease is resistant to a proton pump inhibitor.
[16] The agent according to any of [12] to [14] above, which is used in combination with a medicine selected from proton pump inhibitors, histamine H2 receptor antagonists, gastrointestinal prokinetic agents, oral antacid mixtures, mucosal protective agents and dopamine receptor antagonists.
[17] The agent according to any of [12] to [14] above, which is used in combination with a proton pump inhibitor.
[18] The agent according to [16] or [17] above, wherein the proton pump inhibitor is a medicine selected from omeprazole, esomeprazole, pantoprazole, rabeprazole, tenatoprazole, ilaprazole and lansoprazole.
[19] An agent for suppressing the reflux of gastric acid and/or gastric contents, containing 4-[({6-[isobutyl(1,3-thiazol-2-ylsulfonyl)amino]-2,3-dihydro-1H-inden-5-yl}oxy)methyl]-3-methyl benzoic acid, a salt thereof or a prodrug thereof as an active ingredient.
[20] The agent according to [19] above for the prevention and/or treatment of regurgitation.
[21] A prophylactic or relapse-prevention agent for gastroesophageal reflux disease, containing 4-[({6-[isobutyl(1,3-thiazol-2-ylsulfonyl)amino]-2,3-dihydro-1H-inden-5-yl}oxy)methyl]-3-methyl benzoic acid, a salt thereof or a prodrug thereof as an active ingredient.
[22] The agent according to [21] above, wherein the gastroesophageal reflux disease is non-erosive.
[23] The agent according to [21] or [22] above, which is used in combination with a medicine selected from proton pump inhibitors, histamine H2 receptor antagonists, gastrointestinal prokinetic agents, oral antacid mixtures, mucosal protective agents and dopamine receptor antagonists.
[24] The agent according to [23] above, wherein the proton pump inhibitor is a medicine selected from omeprazole, esomeprazole, pantoprazole, rabeprazole, tenatoprazole, ilaprazole and lansoprazole.
[25] 4-[({6-[Isobutyl(1,3-thiazol-2-ylsulfonyl)amino]-2,3-dihydro-1H-inden-5-yl}oxy)methyl]-3-methyl benzoic acid, a salt thereof or a prodrug thereof for use in the prevention or relapse prevention, treatment and/or amelioration of gastroesophageal reflux disease.
[26] A use of 4-[({6-[isobutyl(1,3-thiazol-2-ylsulfonyl)amino]-2,3-dihydro-1H-inden-5-yl}oxy)methyl]-3-methyl benzoic acid, a salt thereof or a prodrug thereof for manufacturing a prophylactic or relapse-prevention, therapeutic and/or ameliorating agent for gastroesophageal reflux disease.
[27] A method of prevention or relapse prevention, treatment and/or amelioration of gastroesophageal reflux disease, comprising administering a pharmaceutically effective amount of 4-[({6-[isobutyl(1,3-thiazol-2-ylsulfonyl)amino]-2,3-dihydro-1H-inden-5-yl}oxy)methyl]-3-methyl benzoic acid, a salt thereof or a prodrug thereof to a patient with gastroesophageal reflux disease.
[28] 4-[({6-[Isobutyl(1,3-thiazol-2-ylsulfonyl)amino]-2,3-dihydro-1H-inden-5-yl}oxy)methyl]-3-methyl benzoic acid, a salt thereof or a prodrug thereof for use in suppressing hypersensitivity in the esophagus.
[29] A use of 4-[({6-[isobutyl(1,3-thiazol-2-ylsulfonyl)amino]-2,3-dihydro-1H-inden-5-yl}oxy)methyl]-3-methyl benzoic acid, a salt thereof or a prodrug thereof for manufacturing an agent for suppressing hypersensitivity in the esophagus.
[30] A method for suppressing hypersensitivity in the esophagus, comprising administering a pharmaceutically effective amount of 4-[({6-[isobutyl(1,3-thiazol-2-ylsulfonyl)amino]-2,3-dihydro-1H-inden-5-yl}oxy)methyl]-3-methyl benzoic acid, a salt thereof or a prodrug thereof to a patient with gastroesophageal reflux disease.
[31] 4-[({6-[Isobutyl(1,3-thiazol-2-ylsulfonyl)amino]-2,3-dihydro-1H-inden-5-yl}oxy)methyl]-3-methyl benzoic acid, a salt thereof or a prodrug thereof for use in suppressing transient lower esophageal sphincter relaxation.
[32] A use of 4-[({6-[isobutyl(1,3-thiazol-2-ylsulfonyl)amino]-2,3-dihydro-1H-inden-5-yl}oxy)methyl]-3-methyl benzoic acid, a salt thereof or a prodrug thereof for manufacturing an agent for suppressing transient lower esophageal sphincter relaxation.
[33] A method for suppressing transient lower esophageal sphincter relaxation, comprising administering a pharmaceutically effective amount of 4-[({6-[isobutyl(1,3-thiazol-2-ylsulfonyl)amino]-2,3-dihydro-1H-inden-5-yl}oxy)methyl]-3-methyl benzoic acid, a salt thereof or a prodrug thereof to a patient with gastroesophageal reflux disease.
[34] A medicine formed by combining 4-[({6-[isobutyl(1,3-thiazol-2-ylsulfonyl)amino]-2,3-dihydro-1H-inden-5-yl}oxy)methyl]-3-methyl benzoic acid, a salt thereof or a prodrug thereof with an agent selected from proton pump inhibitors, histamine H2 receptor antagonists, gastrointestinal prokinetic agents, oral antacid mixtures, mucosal protective agents and dopamine receptor antagonists.
[35] The medicine according to [34] above, wherein the proton pump inhibitor is a medicine selected from omeprazole, esomeprazole, pantoprazole, rabeprazole, tenatoprazole, ilaprazole and lansoprazole.
[36] A article of manufacture comprising 4-[({6-[Isobutyl(1,3-thiazol-2-ylsulfonyl)amino]-2,3-dihydro-1H-inden-5-yl}oxy)methyl]-3-methyl benzoic acid, a salt thereof or a prodrug thereof, a container containing the compound, and a package insert or a label indicating that the compound can be used to prevent, prevent relapse of, or treat gastroesophageal reflux disease and/or ameliorate a symptom thereof.

### EFFECT OF THE INVENTION

The compound of the present invention suppresses hypersensitivity elicited by acids and stimulation other than acids, and thus is effective in the treatment of GERD or amelioration of typical symptoms such as heartburn and acid regurgitation. The compound of the present invention also has a suppressive action on transient lower esophageal sphincter relaxation, and thus can also suppress the onset or relapse of GERD by suppressing the reflux of gastric acid or stomach contents.

### BRIEF DESCRIPTION OF THE DRAWINGS

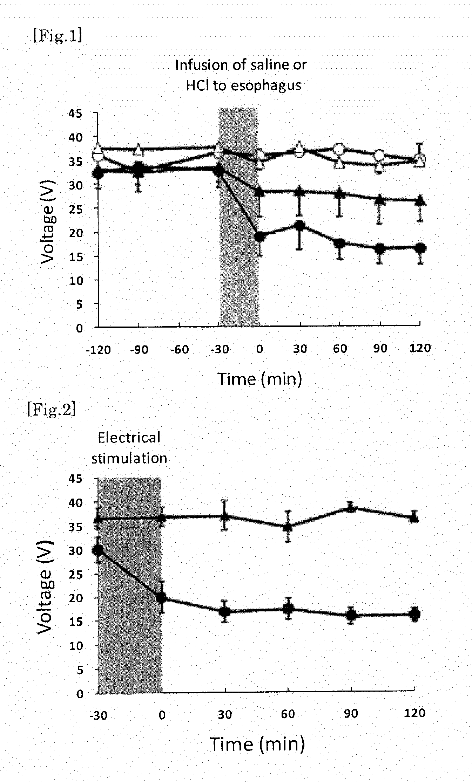

[Fig. 1] Fig. 1 shows the evaluation results of the compound of the present invention in a hypersensitivity model elicited by an acid, wherein the axis of ordinate indicates the voltage (V) of electrical stimulation exhibiting a specific nociceptive behavior, and the end of perfusion of hydrochloric acid or a physiological saline solution into the esophagus is considered as 0-min of time (min) on the axis of abscissa, and open circles (ο) indicate the healthy control group, closed circles (●) indicate the control group, closed triangles (A) indicate the Compound A (0.01 mg/kg)-administered group, and open triangles (Δ) indicate the Compound A (0.1 mg/kg)-administered group.
[Fig. 2] Fig. 2 shows the evaluation results of the compound of the present invention in a hypersensitivity model elicited by electrical stimulation, wherein the axis of ordinate indicates the voltage (V) of electrical stimulation exhibiting a specific nociceptive behavior, and the end of electrical stimulation for 30 minutes is considered as 0-min of time (min) on the axis of abscissa, and closed circles (●) indicate the control group, and closed triangles (A) indicate the Compound A (0.1 mg/kg)-administered group.

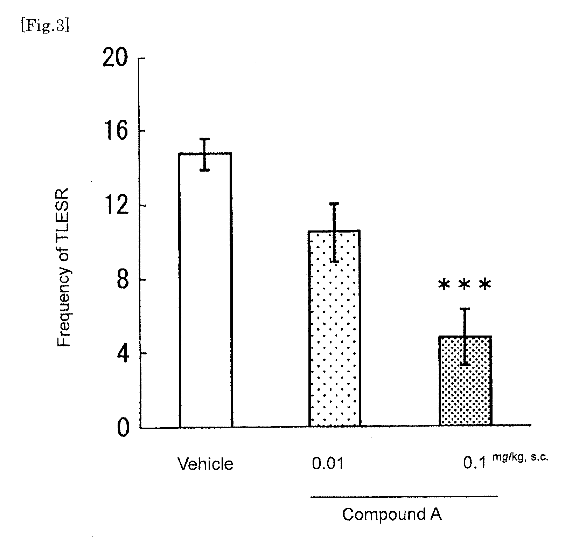

[Fig. 3] Fig. 3 shows the evaluation results of the compound of the present invention in a model of transient lower esophageal sphincter relaxation, wherein the axis of ordinate indicates the frequency of transient lower esophageal sphincter relaxation, and "***" indicates a significant difference (P < 0.01) from the control group (the medium-administered group) by a multiple comparison test by Steel's test.

### MODE FOR CARRYING OUT THE INVENTION

In the present specification, the compound of the present invention means 4-[({6-[isobutyl(1,3-thiazol-2-ylsulfonyl)amino]-2,3-dihydro-1H-inden-5-yl}oxy)methyl]-3-methyl benzoic acid, a salt thereof or a prodrug thereof, and particularly Compound A can be produced by suitably improving the method described in WO 2002/072564 and a known method, e.g. the method described in Synlett 2002, No. 1, 239-242 or Comprehensive Organic Transformations: A Guide to Functional Group Preparations, 2nd Edition (Richard C. Larock, John Wiley & Sons Inc., 1999) and the like, and using the methods in combination.

Salts of the compound of the present invention include acid addition salts (e.g. inorganic salts such as hydrochlorides, hydrobromates, hydroiodides, sulfates, phosphates and nitrates; and organic salts such as acetates, lactates, tartrates, benzoates, citrates, methanesulfonates, ethanesulfonates, benzene sulfonates, toluenesulfonates, isethionates, glucuronates and gluconates), salts of alkali metals (potassium, sodium etc.), salts of alkaline earth metals (calcium, magnesium etc.), ammonium salts or salts of pharmaceutically acceptable organic amines (e.g. tetramethylammonium, triethylamine, methylamine, dimethylamine, cyclopentylamine, benzylamine, phenethylamine, piperidine, monoethanolamine, diethanolamine, tris(hydroxymethyl)aminomethane, lysine, arginine, and N-methyl-D-glucamine), and the like. The compound of the present invention can be converted into a corresponding salt by a known method.

Examples of prodrugs of the compound of the present invention include compounds in which the carboxy group of Compound A is alkyl-esterified, phenyl-esterified, carboxymethyl-esterified, dimethylaminomethyl-esterified, pivaloyloxymethyl-esterified, ethoxycarbonyloxyethyl-esterified, phthalidyl-esterified, (5-methyl-2-oxo-1,3-dioxolen-4-yl)methyl-esterified, cyclohexyloxycarbonylethyl-esterified or methyl-amidated, and these compounds can be produced by known methods. The compound in which the carboxy group of Compound A is alkyl-esterified is preferably methyl 4-({[6-(N-isobutylthiazole-2-sulfonamido)-2,3-dihydro-1H-inden-5-yl]oxy}methyl)-3-methylbenzoate.

In the present specification, the symptoms of GERD mainly include heartburn and acid regurgitation, and these are generally called typical symptoms. Meanwhile, examples of accessory symptoms of GERD include epigastralgia, dysphagia, emesis, eructation, and abdominal distension, and examples of atypical symptoms include chest pain, chronic cough and laryngopharyngeal symptoms.

In the present specification, the amelioration of GERD means that one or more symptoms observed in GERD disappear or are recessive as compared to symptoms before administration of the compound of the present invention.

The compound of the present invention is effective in the amelioration of typical symptoms such as heartburn and/or acid regurgitation in GERD. The compound of the present invention is also effective in the treatment or amelioration of NERD of GERD, and further effective for a patient with GERD or a patient with NERD in which agents used as therapeutic agents for GERD, in particular, proton pump inhibitors are not effective. Furthermore, the compound of the present invention also has a suppressive action on Transient Lower Esophageal Sphincter Relaxation (TLESR: Transient Lower Esophageal Sphincter Relaxation), and thus can suppress the onset or relapse of GERD by suppressing the reflux of gastric acid and stomach contents. The compound of the present invention generally reduces the exposure of gastric acid to the esophagus, and thus is useful in the treatment or amelioration of GERD.

Usually, the compound of the present invention is orally administered in a dose of 10 mg to 300 mg (e.g. 10 mg, 30 mg, 100 mg, 300 mg) per adult per administration once to three times daily. Naturally, the dose varies depending on various conditions such as age, body weight, symptoms, therapeutic effects, administration methods and treatment time. Therefore, in some cases, an amount less than the above-mentioned dose is enough, and in other cases, administration beyond the range is needed.

The compound of the present invention may be administered in combination with a different agent for (1) the complement and/or reinforcement of the effects of prevention or relapse prevention, or treatment or amelioration of gastroesophageal reflux disease by the compound of the present invention, (2) improvements in pharmacokinetics and absorption of the compound of the present invention or a different agent, and a decrease in dose, and/or (3) reduction of side effects of a different agent.

A concomitant drug of the compound of the present invention and a different agent may be administered in the form of a combined agent in which both components are combined in a formulation, or may be administered as different formulations. Administration as different formulations includes coadministration and administration at intervals. For administration at intervals, a different agent may be administered after the compound of the present invention is administered, or the compound of the present invention may be administered after a different agent is administered. The administration methods for each administration may be same or different. Examples of different agents herein include proton pump inhibitors, histamine H2 receptor antagonists, gastrointestinal prokinetic agents, oral antacid mixtures, mucosal protective agents, and dopamine receptor antagonists.

Examples of proton pump inhibitors include omeprazole, esomeprazole, pantoprazole, rabeprazole, tenatoprazole, ilaprazole, and lansoprazole.

Examples of histamine H2 receptor antagonists include cimetidine, ranitidine, famotidine, roxatidine, nizatidine, and lafutidine.

Examples of gastrointestinal prokinetic agents include domperidone, metoclopramide, mosapride, itopride, and tegaserod.

Examples of oral antacid mixtures include Maalox (registered trademark), Aludrox (registered trademark), and Gaviscon (registered trademark).

Examples of mucosal protective agents include polaprezinc, ecabet sodium, rebamipide, teprenone, cetraxate, sucralfate, and plaunotol.

Examples of dopamine receptor antagonists include metoclopramide, domperidone, and levosulpiride.

The weight ratio between the compound of the present invention and a different agent is not particularly restricted.

As different agents, two or more agents of any same type or different types may be administered in combination.

Different agents include not only agents which have been found so far but also agents which will be found in the future based on the above-mentioned mechanism.

When the compound of the present invention is administered independently or administered in combination with a different agent, the compound is used as a solid medicine for internal use or a liquid medicine for internal use for oral administration.

The solid medicines for internal use for oral administration include tablets, pills, capsules, powders, and granules. Capsules include hard capsules and soft capsules. For such solid medicines for internal use, formulations are produced from one or more active substances themselves or after mixing the substances with an activator (e.g. lactose, mannitol, glucose, microcrystalline cellulose, or starch), a binder (e.g. hydroxypropyl cellulose, polyvinylpyrrolidone or magnesium aluminometasilicate), a disintegrator (e.g. calcium carboxymethyl cellulose), a lubricant (e.g. magnesium stearate), a stabilizer, a solubilizing agent (e.g. glutamic acid or aspartic acid) and the like according to usual methods, and used. In addition, the medicines may be coated with coating agents (e.g. white sugar, gelatin, hydroxypropyl cellulose, and hydroxypropyl methylcellulose phthalate) as needed, or coated with two or more layers. Further, capsules of substances which can be absorbed such as gelatin are also included.

The liquid medicines for internal use for oral administration include pharmaceutically acceptable solutions, suspensions, emulsions, syrups, and elixirs. For such liquid medicines, one or more active substances are dissolved, suspended or emulsified in a diluent which is generally used (e.g. purified water, ethanol or a mixed liquid thereof). The liquid medicines may further contain a wetting agent, a suspending agent, an emulsifier, a sweeting agent, a flavoring agent, an aromatic, a preservative, a buffering agent and the like.

The present invention will be described in more detail by the following examples. It should be noted, however, that the present invention is not limited thereto. Various alterations and modifications can be made by those of skill in the art based on the description of the present invention, and these alterations and modifications are also included in the present invention.

### Examples

### Example 1: Evaluation in hypersensitivity model evoked by acid

Cynomolgus monkeys (aged 4 to 6 years, female, Japan Laboratory Animals, Inc., no history of administration) were acclimated to a monkey chair for 2 days or more, and were fasted for 12 hours or more. After that, on the day of experiment, the monkeys were immobilized in the monkey chair after the measurement of body weight, and were acclimated for another one hour or more. An electrostimulation catheter was inserted from a nostril, and electrical stimulation was applied at the position approximately 10 cm above the cardiac part of stomach (the upper esophagus). The voltage was increased in increments of approximately 2 V/sec, and the voltage (V) exhibiting a specific nociceptive behavior (an escape reflex behavior to escape from a cage) in an individual was obtained. This was carried out three times every 2 to 3 minutes at each evaluation time point, and the average value was used as a nociceptive threshold at each time point. Saline or hydrochloric acid (1.5 N) was perfused from the position approximately 3 cm above the cardiac region (the lower esophagus) to the stomach at an injection rate of 160 mL/h for 30 minutes. The nociceptive thresholds were measured before and after perfusion of saline in the esophagus, before perfusion of hydrochloric acid in the esophagus and at 0, 30, 60, 90 and 120 minutes after perfusion of hydrochloric acid in the esophagus. The vehicle or Compound A (0.01 and 0.1 mg/kg, respectively) was subcutaneously administered 30 minutes before the start of the perfusion of hydrochloric acid.

The number of animals in each group were as follows; 3 animals in the healthy control group (the group in which perfusion of saline, administration of vehicle, and perfusion of saline were carried out in a sequential order), 5 animals in the control group (the group in which perfusion of saline, administration of vehicle, and perfusion of hydrochloric acid were carried out in a sequential order) and the 0.01 mg/kg Compound A group (the group in which perfusion of saline, administration of Compound A and perfusion of hydrochloric acid were carried out in a sequential order) and 7 animals in the 0.1 mg/kg Compound A group (the group in which perfusion of saline, administration of Compound A and perfusion of hydrochloric acid were carried out in a sequential order). The end of the perfusion of hydrochloric acid (in the control group and the Compound A-administered group) or saline (in the healthy control group) in the esophagus for 30 minutes was considered as the base time (0 min), and perfusion of saline in the esophagus in all test groups was carried out between 90 and 120 minutes before the base time.

The nociceptive threshold (V) was used as the endpoint. The endpoint was assessedby comparing the nociceptive thresholds before and after perfusion of hydrochloric acid, the nociceptive threshold of the vehicle-administered group at each time point and the nociceptive threshold of the Compound A-administered group at each time point.

In addition, after the end of the experiment, the entire periphery of the lower esophageal tissue approximately 3 cm above the cardiac region in which the acid was perfused was extracted, and was immersed in a 4% paraformaldehyde phosphate buffer to produce a paraffin embedded block. A paraffin section with a thickness of 4 µm was produced from the paraffin embedded block, and put on a MAS coated slide glass (MATSUNAMI). Hematoxylin eosin staining was carried out, and the section was observed by microscopy.

### [Results]

As shown in Fig. 1, the nociceptive thresholds of the healthy control group were not changed at all time points, and were nearly constant. About the nociceptive threshold of the control group, the nociceptive threshold decreased immediately after hydrochloric acid perfusion, and was maintained up to 120 minutes after hydrochloric acid perfusion. On the other hand, about the nociceptive threshold of the Compound A-administered groups, decrease in the nociceptive thresholds observed after hydrochloric acid perfusion in the control group were dose-dependently suppressed, and in the 0.1 mg/kg Compound A-administered group, the nociceptive threshold was suppressed to a normal level. In addition, the suppressive action was maintained even at 120 minutes after hydrochloric acid perfusion. Furthermore, in the esophageal tissue in which the acid was perfused under the experimental conditions, damage and inflammation images of the esophageal mucosal tissue were observed.

From the above results, it was suggested that the compound of the present invention is effective in the treatment or amelioration of GERD symptoms caused by the reflux of gastric acid, particularly heartburn and acid regurgitation accompanied by esophageal mucosal damages.

### Example 2: Evaluation in hypersensitivity model evoked by electrical stimulation

Cynomolgus monkeys (aged 4 to 6 years, female, Japan Laboratory Animals, Inc., no history of administration) were acclimated to a monkey chair for 2 days or more, and were fasted for 12 hours or more. After that, on the day of experiment, the monkeys were immobilized in the monkey chair after the measurement of body weight, and were acclimated for another one hour or more. An electrostimulation catheter was inserted from a nostril, and electrical stimulation was applied at the position approximately 10 cm above the cardiac part of stomach (the upper esophagus). The voltage was increased in increments of approximately 2 V/sec, and the voltage (V) exhibiting a specific nociceptive behavior (an escape reflex behavior to escape from a cage) in an individual was obtained. This was carried out three times every 2 to 3 minutes at each evaluation time point, and the average value was used as a nociceptive behavior threshold at each time point. Hypersensitivity was induced by continuous electrical stimulation to the position approximately 3 cm above the cardiac region (the lower esophagus). That is, an electrostimulation catheter was inserted from a nostril, and electrical stimulation was applied at the position of the lower esophagus for 30 minutes. The vehicle or Compound A (0.1 mg/kg) was subcutaneously administered 30 minutes before the beginning of eliciting by electrical stimulation. The number of animals was 3 in each group.

The nociceptive threshold (V) was used as the endpoint. The endpoint was assessed by comparing the nociceptive thresholds before and after continuous electrical stimulation, the nociceptive threshold of the vehicle-administered group at each time point and the nociceptive threshold of the Compound A-administered group at each time point.

In addition, after the end of the experiment, the entire periphery of the lower esophagus tissue approximately 3 cm above the cardiac region in which electrical stimulation was carried out was extracted, and was immersed in a 4% paraformaldehyde phosphate buffer to produce a paraffin embedded block. A paraffin section with a thickness of 4 µm was produced from the paraffin embedded block, and put on a MAS coated slide glass (MATSUNAMI). Hematoxylin eosin staining was carried out, and the section was observed by microscopy.

### [Results]

As shown in Fig. 2, about the nociceptive threshold of the control group (the group in which administration of a medium and continuous electrical stimulation were carried out in a sequential order), the nociceptive threshold decreased immediately after eliciting and was maintained up to 120 minutes after continuous electrical stimulation. On the other hand, about the nociceptive threshold of the Compound A-administered group (the group in which administration of Compound A and continuous electrical stimulation were carried out in a sequential order), a decrease in the nociceptive threshold observed in the control group after continuous electrical stimulation was suppressed to a normal level. In addition, the suppressive action was maintained even at 120 minutes after continuous electrical stimulation. Furthermore, in the esophageal tissue in which continuous electrical stimulation was carried out under the experimental conditions, damage and inflammation images of the esophageal mucosal tissue were not observed.

From the above results, it was suggested that the compound of the present invention is effective in the treatment or amelioration of GERD which is not caused by inflammation accompanied by the reflux of gastric acid or stomach contents, particularly NERD.

### Example 3: Evaluation of penetration into the center

Cynomolgus monkeys (aged 4 to 6 years, female, Japan Laboratory Animals, Inc., no history of administration) were immobilized in a monkey chair, and Compound A was subcutaneously administered thereto (0.1 mg/kg), followed by collecting blood at 180 minutes after administration. The blood samples were centrifuged at 10000 g at 4°C for 3 minutes to collect plasma. Next, the cerebrospinal fluid was collected under anesthesia by intravenous administration of pentobarbital sodium, and the lower esophagus, the upper esophagus, the dorsal root ganglion, the spinal cord and the brain stem were then collected. Furthermore, the dorsal root ganglion and the spinal cord were collected from the first to sixth thoracic spinal cord, which controls esophageal nerves. To the collected tissue, 4 times the amount of saline per wet weight of each tissue was added, followed by homogenization using Physcotron to obtain each tissue homogenate. The number of animals was 3.

The concentration of Compound A in each tissue was measured by a LC/MS/MS method.

To the samples for 50 µL of a calibration curve and test samples of Compound A, 1 mL of ultrapure water and an internal standard substance were added under ice cold conditions, followed by stirring. To a solid phase extraction cartridge (OASIS MAX 30 mg/l cc, Waters Corporation) which had been conditioned in advance, each sample was loaded and washing was carried out, followed by centrifugation, and a cleaning liquid was discharged from the cartridge for drying. Further, elution was carried out with 1 mL of an acetonitrile/acetic acid mixed liquid (49 : 1), and the eluate was concentrated and dried under a current of nitrogen (40°C or less). The residue was redissolved in 200 µL of a methanol/0.1 vol% acetic acid solution (70 : 30) to obtain a sample for the measurement of LC/MS/MS.

The analysis by LC/MS/MS was carried out under the following conditions.

### [LC conditions]

Measuring device: Nanospace 3033 (Shiseido Co., Ltd.)
Analysis column: SunFire C18 3.5 µm (2.1 mm i.d. X 100 mm, 3.5 µm, Waters Corporation)
Mobile phase: Acetonitrile/0.1 vol% acetic acid solution (70 : 30)
Cleaning liquid for cleaning port (one component): 2-propanol
Cleaning liquid for cleaning port (two components): Mobile phase
[acetonitrile/0.1 vol% acetic acid solution (70 : 30)]
Flow rate: 0.2 mL/min
Column temperature: 35°C
Sample temperature: 4°C
Analysis time: 13 minutes

### [MS/MS conditions]

Measuring device: API4000 (AB SCIEX)
Ion source: ESI
Scan type: MRM
Polarity: Negative
Temperature: 650°C
Monitoring ion: Q1: 499.0 (m/z), Q3: 147.3 (m/z)

Using the peak area ratio (standard substance/internal standard substance) obtained by the measurement of a sample for a calibration curve, the calibration curve (y = aX + b, Y: Peak area ratio, X: Concentration) was made from the single regression line by the least squares method, and the peak area ratios of test samples were applied to the calibration curve to calculate a measured value.

### [Results]

At 180 minutes after administration of Compound A, the average concentrations in plasma, the cerebrospinal fluid, the lower esophagus, the upper esophagus, the dorsal root ganglion, the spinal cord and the brain stem tissue were 3.79, 0.07, 0.25, 0.28, 0.62, 0.19 and 0.22 ng/mL, respectively. The percentages to the concentration in plasma were 1.9, 6.7, 7.3, 16.3, 5.1 and 5.7%, respectively.

### Example 4: Evaluation of action on transient lower esophaegeal sphincter relaxation (TLESR) (1)

Cynomolgus monkeys (aged 4 to 6 years, female, Japan Laboratory Animals, Inc., no history of administration) are acclimated to a monkey chair for 2 days or more, and are fasted for 12 hours or more. After that, on the day of experiment, the monkeys are immobilized in the monkey chair after the measurement of body weight, and are acclimated for another one hour or more. A high-fat diet/air injection catheter and a sensor catheter for measuring pressure are inserted from a nostril. A high-fat diet is injected into the stomach so that the final volume will be 80 mL, and air is then injected up to a pressure in the stomach of 10 mmHg, and maintained. The pressure in the esophagus is measured by a sensor catheter in the stomach, the lower esophageal sphincter region and the position approximately 3 cm above the lower esophageal sphincter. The frequency of TLESR is measured for 45 minutes from the injection of high-fat diet before and after the subcutaneous administration of Compound A (0.1 mg/kg) to the end of ventilation.

### Example 5: Evaluation of action on Transient Lower Esophageal Sphincter Relaxation (TLESR) (2)

Cynomolgus monkeys (aged 4 to 6 years, female, Japan Laboratory Animals, Inc., no history of administration) were acclimated to a monkey chair for 2 days or more, and were fasted for 12 hours or more. After that, on the day of experiment, the monkeys were immobilized in the monkey chair after the measurement of body weight, and was acclimated for another one hour or more. A high-fat diet/air injection catheter and a sensor catheter for measuring pressure were inserted from a nostril and the oral cavity. A high-fat diet was injected into the stomach so that the final volume would be 80 mL, and air was then injected up to a pressure in the stomach of 20 mmHg, and maintained. The pressure in the esophagus was measured by a sensor catheter in the lower esophageal sphincter region and the positions approximately 2, 3 and 6 cm above the lower esophageal sphincter. Compound A was subcutaneously administered (0.01 and 0.1 mg/kg), and the injection of high-fat diet and ventilation were carried out at 30 minutes after subcutaneous administration. The frequency of TLESR was measured for 45 minutes from the injection of high-fat diet to the end of ventilation. Here, TLESR was measured considering that the relaxation response of the lower esophageal sphincter region was longer by 0.5 seconds and was not elicited by peristaltic motion occurring in the esophagus, and the rate of reduction of pressure was greater than 0.5 seconds, and measured. The number of animals in the groups (the control group (the vehicle-administered group), the 0.01 mg/kg Compound A-subcutaneously-administered group and the 0.1 mg/kg Compound A-subcutaneously-administered group) was 4, respectively.

### [Results]

TLESR did not occur before the injection of high-fat diet and the injection of air at all, however, occurred by the injection of high-fat diet and the injection of air. As shown in Fig. 3, the frequency of TLESR in the Compound A-administered group dose-dependently decreased as compared to the frequency of TLESR in the control group (the vehicle-administered group).

As in the above results, it was suggested that Compound A suppresses the occurrence of TLESR after the injection of high-fat diet and air, and thus the compound of the present invention can suppress the onset or relapse of GERD by suppressing the reflux of gastric acid and gastric contents, and further is effective in the treatment or amelioration of GERD.

### INDUSTRIAL APPLICABILITY

The compound of the present invention is useful in the prevention or relapse prevention, treatment or amelioration of GERD.

## Claims

1. An agent for suppressing transient lower esophageal sphincter relaxation, comprising 4-[({6-[isobutyl(1,3-thiazol-2-ylsulfonyl)amino]-2,3-dihydro-1H-inden-5-yl}oxy)methyl]-3-methyl benzoic acid, a salt thereof or a prodrug thereof as an active ingredient.

2. The agent according to claim 1, which reduces the frequency of transient lower esophageal sphincter relaxation.

3. An agent for suppressing the reflux of gastric acid and/or gastric contents, comprising 4-[({6-[isobutyl(1,3-thiazol-2-ylsulfonyl)amino]-2,3-dihydro-1H-inden-5-yl}oxy)methyl]-3-methyl benzoic acid, a salt thereof or a prodrug thereof as an active ingredient.

4. An agent for preventing or relapse-preventing gastroesophageal reflux disease, comprising 4-[({6-[isobutyl(1,3-thiazol-2-ylsulfonyl)amino]-2,3-dihydro-1H-inden-5-yl}oxy)methyl]-3-methyl benzoic acid, a salt thereof or a prodrug thereof as an active ingredient.

5. An agent for treating or ameliorating gastroesophageal reflux disease, comprising 4-[({6-[isobutyl(1,3-thiazol-2-ylsulfonyl)amino]-2,3-dihydro-1H-inden-5-yl}oxy)methyl]-3-methyl benzoic acid, a salt thereof or a prodrug thereof as an active ingredient.

6. The agent according to claim 5, wherein the gastroesophageal reflux disease is non-erosive.

7. The agent according to claim 5 or 6, wherein the symptoms of the gastroesophageal reflux disease are heartburn and/or acid reflux.

8. The agent according to any one of claims 5 to 7, wherein the gastroesophageal reflux disease is resistant to a proton pump inhibitor.

9. The agent according to any one of claims 5 to 8, which is used in combination with a medicine selected from proton pump inhibitors, histamine H2 receptor antagonists, gastrointestinal prokinetic agents, oral antacid mixtures, mucosal protective agents and dopamine receptor antagonists.

10. The agent according to claim 9, wherein the proton pump inhibitor is a medicine selected from omeprazole, esomeprazole, pantoprazole, rabeprazole, tenatoprazole, ilaprazole and lansoprazole.
